(19) Europäisches Patentamt / European Patent Office / Office européen des brevets

(11) **EP 1 281 384 B1**

(12) **FASCICULE DE BREVET EUROPEEN**

(45) Date de publication et mention
de la délivrance du brevet:
**18.03.2009 Bulletin 2009/12**

(51) Int Cl.:
*A61K 8/04* *(2006.01)*     *A61K 8/19* *(2006.01)*
*A61K 8/37* *(2006.01)*     *A61K 8/73* *(2006.01)*
*A61K 8/92* *(2006.01)*     *A61Q 1/10* *(2006.01)*

(21) Numéro de dépôt: **02291690.2**

(22) Date de dépôt: **05.07.2002**

(54) **Mascara comprenant des particules solides**

Mascara, die feste Teilchen enthält

Mascara comprising solid particles

(84) Etats contractants désignés:
**AT BE BG CH CY CZ DE DK EE ES FI FR GB GR
IE IT LI LU MC NL PT SE SK TR**

(30) Priorité: **16.07.2001 FR 0109505**

(43) Date de publication de la demande:
**05.02.2003 Bulletin 2003/06**

(73) Titulaire: **L'ORÉAL**
**75008 Paris (FR)**

(72) Inventeurs:
• **Auguste, Frédéric**
**Chevilly-Larue (FR)**
• **Tournilhac, Florence**
**75011 Paris (FR)**

(74) Mandataire: **Boulard, Denis**
**L'OREAL - D.I.P.I.**
**25-29 Quai Aulagnier**
**92600 Asnières (FR)**

(56) Documents cités:
**EP-A- 0 557 196          EP-A- 1 048 282
EP-A- 1 064 920          EP-A- 1 082 953
EP-A- 1 108 415          EP-A- 1 201 222
FR-A- 2 794 970**

Remarques:
Le dossier contient des informations techniques
présentées postérieurement au dépôt de la demande
et ne figurant pas dans le présent fascicule.

**Description**

La présente invention a pour objet une composition cosmétique de revêtement des fibres kératiniques, notamment des cils ou des cheveux, comprenant des particules solides et un polymère adhérent, et son utilisation pour recourber les fibres kératiniques. La composition est destinée aux fibres kératiniques sensiblement longitudinales d'êtres humains comme les cils ou les cheveux ou bien encore aux faux-cils ou aux postiches comme les perruques. Plus spécialement, la composition est destinée au revêtement des cils.

La composition peut être une composition de maquillage, encore appelé mascara, une composition à appliquer sur un maquillage, dite encore top-coat, ou bien encore une composition de traitement des fibres kératiniques, notamment des cils. Plus spécialement, la composition est un mascara.

Le but de la présente invention est de proposer une composition de revêtement des cils conduisant après application, à un revêtement conférant un bon recourbement des cils.

Les inventeurs ont découvert qu'un tel revêtement des cils pouvait être obtenu en utilisant des particules solides particulières associées à un polymère adhérent.

De façon plus précise, l'invention a pour objet une composition de revêtement des fibres kératiniques, notamment des cils, sous forme de dispersion cire-dans-eau comprenant, dans un milieu aqueux cosmétiquement acceptable, une fraction non volatile comprenant :

- un polymère apte à adhérer sur les matières kératiniques tel que défini dans la revendication 1,
- des premières particules solides à 25 °C comprenant un premier matériau comprenant comprenant une cire ayant un point de fusion inférieur à 77 °C et une dureté supérieure ou égale à 6,5 MPa,
- et éventuellement des deuxièmes particules solides comprenant un deuxième matériau différent du premier matériau, les deuxièmes particules solides n'étant pas aptes à coalescer à une température inférieure ou égale à 40 °C,

les premières particules solides et, le cas échéant, les deuxièmes particules solides étant présentes dans la composition en une teneur telle que la fraction volumique des premières particules solides et, le cas échéant, des deuxièmes particules solides est supérieure ou égale à 70 % du volume total de la fraction non volatile de la composition, et, le cas échéant, la fraction volumique des premières particules solides est supérieure ou égale à 25 % du volume total des premières particules solides et des deuxièmes particules solides.

L'invention a également pour objet un procédé de maquillage ou de soin non thérapeutique des fibres kératiniques, notamment des cils, comprenant l'application sur les fibres kératiniques d'une composition telle que définie précédemment.

L'invention a aussi pour objet l'utilisation d'une composition telle que définie précédemment pour recourber les cils.

L'invention a encore pour objet l'utilisation de premières particules solides à 25 °C comprenant un premier matériau d'une cire ayant un point de fusion inférieur à 77 °C et une dureté supérieure ou égale à 6,5 MPa, et éventuellement de deuxièmes particules solides d'un deuxième matériau différent du premier matériau, les deuxièmes particules solides n'étant pas aptes à coalescer à une température inférieure ou égale à 40 °C, dans une composition de revêtement des fibres kératiniques, notamment un mascara, sous forme de dispersion cire-dans-eau comprenant, dans un milieu aqueux cosmétiquement acceptable, une fraction non volatile comprenant un polymère apte à adhérer sur les matières kératiniques tel que défini dans la revendication 1, et les dites premières particules solides et, le cas échéant, lesdites deuxièmes particules solides, les premières et, le cas échéant, les deuxièmes particules solides étant présentes dans la composition en une teneur telle que la fraction volumique des premières particules solides et, le cas échéant, des deuxièmes particules solides est supérieure ou égale à 70 % du volume total de la fraction non volatile de la composition, et, le cas échéant, la fraction volumique des premières particules solides est supérieure ou égale à 25 % du volume total des premières particules solides et des deuxièmes particules solides, pour recourber les matières kératiniques, notamment les cils.

On entend par particules solides des particules qui sont à l'état solide à 25 °C et à pression atmosphérique.

On entend par fraction non volatile de la composition l'ensemble des constituants présents dans la composition qui ne sont pas volatiles. On entend par composé volatile un composé qui, pris isolément, a une pression de vapeur non nulle, à température ambiante (25 °C) et pression atmosphérique, allant en particulier de $10^{-2}$ à 300 mm de Hg (1,33 Pa à 40.000 Pa) et de préférence supérieure à 0,3 mm de Hg (40 Pa).

La fraction non volatile de la composition correspond en fait au mélange des constituants restant sur les cils après le séchage complet du mascara appliqué sur les cils.

Pour obtenir un bon recourbement des cils, la composition selon l'invention comprend des particules solides, dites premières particules solides, comprenant (en particulier formé de) un premier matériau choisi parmi les cires, appelée cires dures, ayant un point de fusion inférieur à 77 °C et une dureté supérieure ou égale à 6,5 MPa.

Par "cire", on entend au sens général de la présente invention, un composé gras lipophile, solide à température ambiante (25 °C) et pression atmosphérique (760 mm de Hg, soit $10^5$ Pa), à changement d'état solide/liquide réversible, ayant une température de fusion supérieure à 30 °C et pouvant aller jusqu'à 200 °C, notamment jusqu'à 120 °C (la cire formant les premières particules solides décrites précédemment correspond à une cire particulière ayant les caractéristiques de point de fusion et de dureté mentionnées).
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Avantageusement, la cire dure a un point de fusion inférieur à 60 °C, notamment allant de 30 °C à 59 °C, de préférence allant de 35 °C à 59 °C, et mieux allant de 40 °C à 50 °C.

De préférence, la cire dure peut avoir une dureté allant de 6,5 MPa à 20 MPa, notamment allant de 6,5 à 15 MPa, en particulier allant de 6,5 à 12 MPa, de préférence allant de 9,7 à 20 MPa, avantageusement allant de 9,7 à 15 MPa, et mieux allant de de 9,7 à 12 à MPa.

Selon la présente demande, la dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Comme cire dure répondant aux critères définis précédemment, on peut utiliser la cire de Candellila, la cire de jojoba hydrogénée, la cire de sumac, la cérésine, le stéarate d'octacosanyle, le stéarate de tétracontanyle, la cire de Shellac, le fumarate de béhényle, le tétrastéarate de di-(triméthylol-1,1,1 propane) vendu sous la dénomination « HEST 2T-4S » par la société HETERENE, le tétrabéhénate de di(triméthylol-1,1,1 propane) vendue sous la dénomination HEST 2T-4B par la société HETERENE, les ozokerites comme celle vendue sous la dénomination « OZOKERITE WAX SP 1020 P » par la société STRAHL & PITSCH

On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A- 2792190.

Avantageusement, la cire dure peut être choisie parmi la cire d'olive obtenue par hydrogénation d'huile d'olive estérifiée avec l'alcool stéarylique vendue sous la dénomination PHYTOWAX Olive 18 L 57 de la société SOPHIM et le tétrastéarate de di-(triméthylol-1,1,1 propane).

Avantageusement, les dites premières particules de cire dure peuvent avoir une taille moyenne allant de 50 nm à 50 $\mu$m, et de préférence de 100 nm à 10 $\mu$m.

Les premières particules de cire dure peuvent être présente dans la composition en une teneur allant de 1,25 % à 50 % en poids, par rapport au poids total de la composition, avantageusement allant de 5 % à 40 % en poids, et de préférence allant de 10 % à 25 % en poids.

La composition selon l'invention peut comprendre, en plus des premières particules solides décrites précédemment, d'autres particules solides, appelées deuxièmes particules, différentes des premières particules solides.
Ces deuxièmes particules correspondent aux particules solides à 25 °C de tout matériau, différent des premières particules, restant sous forme de particules individualisées, ou éventuellement collées mais qui conservent dans ce cas leur état de particule individuelle (ces particules collées ne sont pas coalescées à une température inférieure ou égale à 40 °C).

En particulier, les deuxièmes particules solides peuvent comprendre des particules choisies parmi :

- des particules solides à 25 °C, appelées deuxièmes particules solides primaires, comprenant (en particulier formé de) un matériau cristallin ou semi-cristallin solide à température ambiante (25 °C) présentant une transition de phase de premier ordre, de fusion ou de combustion, supérieure à 100 °C ;
- et/ou des particules solides à 25 °C, appelées deuxièmes particules solides secondaires, comprenant un matériau (en particulier formé de) amorphe ayant une température de transition vitreuse supérieure ou égale à 60 °C,
- et/ou d'autres particules solides à 25 °C, appelées deuxièmes particules solides tertiaires, différentes desdites deuxièmes particules primaires et secondaires décrites précédemment
- et leurs mélanges.

Les deuxièmes particules solides primaires sont des particules solides comprenant un matériau, appelé premier matériau, cristallin ou semi-cristallin solide à température ambiante (25 °C) présentant une transition de phase de premier ordre, de fusion ou de combustion, supérieure à 100 °C, de préférence supérieure à 120 °C, et mieux supérieure à 150 °C. La température de fusion ou de combustion du premier matériau peut être mesurée selon la norme ASTM E794-98.

Par "matériau semi-cristallin", on entend au sens de l'invention, un matériau, notamment un polymère, comportant une partie cristallisable et une partie amorphe présentant une température de changement de phase réversible du premier ordre, en particulier de fusion (transition solide-liquide).

Avantageusement, le matériau cristallin ou semi-cristallin présente une dureté Vickers supérieure ou égale à 10, notamment allant de 10 à 7500, de préférence supérieure ou égale à 200, notamment allant de 200 à 7500, et mieux supérieure ou égale à 400, notamment allant de 400 à 7500.

La dureté VICKERS (HV) est déterminée en appliquant sur le matériau un pénétromètre en forme de pyramide à base carrée, à l'aide d'une charge P. On mesure ensuite la dimension moyenne d'une diagonale de l'empreinte carrée obtenue avec le pénétromètre.

La dureté VICKERS (HV) est alors calculée par la relation :

$$HV = \frac{1854,4 \times P}{d^2}$$

d = diagonale moyenne en $\mu$m

P = charge appliquée en g

d = diagonale moyenne en $\mu$m

P = charge appliquée en g

La mesure de la dureté VICKERS peut être effectuée à l'aide du microduromètre M 400 g 2 de la société LECO.

Le premier matériau desdites deuxièmes particules primaires peut être un matériau minéral qui peut être choisi parmi la silice, le verre, le diamant, le cuivre, le nitrure de bore, les céramiques, les micas, les oxydes métalliques, notamment les oxydes de fer comme l'oxyde de fer noir, l'oxyde de fer rouge, l'oxyde de fer jaune, les oxydes de titane, l'alumine, ou bien encore un polymère comme les polyamides par exemple le nylon, et leurs mélanges.

Les dites deuxièmes particules primaires peuvent être des particules pleines, ou bien encore des particules creuses. Par exemple, on peut utiliser la silice creuse vendue sous la dénomination « SUNSIL-130 » par la société SUNJIN CHEMICAL.

Selon un premier mode de réalisation de la composition selon l'invention, lesdites deuxièmes particules primaires sont formées essentiellement dudit premier matériau défini précédemment.

Selon un deuxième mode de réalisation de la composition selon l'invention, ledistes deuxièmes particules solides primaires comprennent, voire sont formées essentiellement de, au moins deux premiers matériaux différents. C'est par exemple le cas des micas recouverts d'oxyde de titane ou d'oxyde de fer.

Selon un troisième mode de réalisation de la composition selon l'invention, lesdites deuxièmes particules primaires comprennent au moins ledit premier matériau, et au moins un matériau additionnel, différent dudit premier matériau, ledit premier matériau formant la surface desdites premières particules. Pour ces particules solides, ledit premier matériau ayant les caractéristiques décrites précédemment se trouve à la surface desdites deuxièmes particules solides primaires, ces dernières comprenant un matériau additionnel recouvert par le premier matériau.

Avantageusement, les dites deuxièmes particules solides primaires peuvent avoir une taille moyenne allant de 5 nm à 50 $\mu$m, et de préférence de 20 nm à 50 $\mu$m.

Les deuxièmes particules solides secondaires comprennent (en particulier sont formées de) un matériau amorphe, en particulier un polymère, ayant une température de transition vitreuse supérieure ou égale à 60 °C (notamment allant de 60 °C à 800 °C), avantageusement supérieure ou égale à 80 °C (notamment allant de 80 °C à 700 °C), et de préférence supérieure ou égale à 100 °C (notamment allant de 100 °C à 500 °C). La température de transition vitreuse peut être mesurée par DSC (Differential Scanning Calorimetry) selon la norme ASTM D3418-97.

Comme matériau amorphe, on peut utiliser un polymère non filmogène à une température inférieure ou égale à 40 °C et ayant une température de transition vitreuse telle que décrite précédemment.

On entend par « polymère non filmogène » un polymère qui n'est pas apte à former, à lui seul ou en présence d'un agent auxiliaire de filmification, un film continu et adhérent sur un support, notamment sur les matières kératiniques, à une température inférieure ou égale à 40 °C.

On entend par agent auxiliaire de filmification les agents plastifiants et les agents de coalescence connus de l'homme du métier pour favoriser la filmification des polymères.

Comme polymère amorphe ayant une température de transition vitreuse supérieure ou égale à 60 °C, on peut utiliser des polymères radicalaires ou les polycondensats ayant cette température de transition vitreuse définie.

Comme polymère radicalaire, on peut citer :

- les polymères d'éthylène, notamment de cycloéthylène, de naphtyléthylène ;
- les polymères de propylène, notamment d'hexafluoropropylène ;
- les polymères acryliques, notamment les polymères d'acide acrylique, d'acrylate de diméthyl-adamanthyl, de chloroacrylate ;
- les polymères d'acrylamide ;
- les polymères de (méth)acrylonitrile ;
- les polymères d'acétylstyrène, de carboxystyrène, de chlorométhylstyrène ;

Comme polycondensats, on peut citer les polycarbonates, les polyuréthanes, les polyesters, les polyamides, les polysulfones, les polysulfonamides, les carbohydrates comme l'amylose triacétate.

Les deuxièmes particules solides secondaires peuvent être des particules pleines ou des particules creuses.

Selon un premier mode de réalisation, les deuxièmes particules solides secondaires sont formées essentiellement dudit matériau amorphe décrit précédemment.

Selon un deuxième mode de réalisation, les deuxièmes particules solides secondaires comprennent au moins ledit premier matériau amorphe et au moins un matériau additionnel, différent du premier matériau amorphe, ledit premier matériau amorphe formant la surface, ou l'écorce, desdites deuxièmes particules solides secondaires et le matériau additionnel formant l'intérieur, ou le coeur, desdites deuxièmes particules solides secondaires.

Le matériau additionnel peut être par exemple un polymère additionnel ayant une température de transition vitreuse inférieure à 60 °C, et de préférence inférieure à 45 °C.

Ainsi, les deuxièmes particules solides secondaires peuvent être par exemple des particules coreshell de polymères, comprenant une partie externe (c'est-à-dire une écorce) formée du premier matériau amorphe ayant une température de transition vitreuse supérieure ou égale à 60 °C et comprenant une partie interne (c'est-à-dire un coeur) ayant une température de transition vitreuse inférieure à 60 °C.

Avantageusement, la teneur du premier matériau amorphe dans les deuxièmes particules solides secondaires est telle que la fraction volumique du premier matériau est supérieure ou égale à 10 %, et de préférence supérieure ou égale à 30 %, en volume du volume total des deuxièmes particules solides secondaires.

Les deuxièmes particules solides primaires peuvent avoir une taille moyenne allant de 10 nm à 50 $\mu$m, et de préférence allant de 20 nm à 1 $\mu$m.

Comme deuxièmes particules solides primaires, on peut utiliser les dispersions aqueuses de polymère non filmogène vendues sous les dénominations « JONCRYL® SCX 8082 », « JONCRYL® 90 » par la société JOHNSON POLYMER, « NEOCRYL® XK 52 » par la société AVECIA RESINS, « RHODOPAS® 5051 » par la société RHODIA CHIMIE.

Toutes les constituants présents dans la composition selon l'invention se trouvant à l'état de particules solides à 25 °C et qui ne coalescent pas à une température inférieure ou égale à 40 °C, à eux seuls ou en présence des autres constituants présents dans la composition, sont considérés comme étant soit des premières particules solides ou soit des deuxièmes particules solides selon les définitions décrites précédemment.

Ainsi, par exemple, les deuxièmes particules peuvent être en un matériau choisi parmi les cires additionnelles, différente de la cire décrite précédemment, les charges, les polymères différents du matériau amorphe présent dans les deuxièmes particules solides secondaires décrites précédemment.

Les additifs décrits ci-après, lorsqu'ils sont sous la forme de particules solides à 25 °C, sont considérés comme étant des deuxièmes particules solides décrites précédemment lorsque ces additifs possèdent les caractéristiques correspondantes définies précédemment.

En particulier, le polymère adhérent présent dans la composition selon l'invention peut être sous la forme de particules solides. Dans ce cas, ces particules sont considérées comme étant des particules solides telles que définies précédemment si ce polymère possède les caractéristiques correspondantes définies précédemment.

Dans la composition selon l'invention, les dites premières et, le cas échéant, les deuxièmes particules solides sont présentes en une teneur telle que la fraction volumique des premières particules solides et, le cas échéant, des deuxièmes particules solides est supérieure ou égale à 70 % (notamment va de 70 % à 95 %) du volume total de lafraction non volatile de la composition.

On entend par « fraction volumique des premières particules solides et, le cas échéant, des deuxièmes particules solides» le pourcentage du volume total de toutes les premières particules solides et, le cas échéant, de toutes les deuxièmes particules solides présentent dans la fraction non volatile de la composition, par rapport au volume total de tous les composés de la fraction non volatile de la composition.

La fraction volumique (FV) de particules solides présente dans la fraction non volatile de la composition est égale au pourcentage du volume total V desdites particules divisé par le volume total V' de la fraction non volatile de la composition.

Le volume V de particules solides est égal à la masse m desdites particules solides dans la composition divisé par la masse volumique Mv des particules. La masse volumique est calculée selon la méthode décrite ci-après.

$$\text{Fraction volumique : } FV = 100 \times V / V' \quad \text{et} \quad V = m / Mv$$

Le volume total V' de la fraction non volatile de la composition est calculé en additionnant le volume de chaque constituant non volatil présent dans la composition.

Avantageusement, lorsque la composition comprend des deuxièmes particules telles que définies précédemment, les premières particules sont présentes dans la composition en une teneur telle que la fraction volumique des premières particules solides est supérieure ou égale à 30 % de la fraction volumique totale des premières et deuxièmes particules solides, notamment allant de 30 % à 100 %, avantageuseument supérieure ou égale à 40 %, notamment allant de 40 % à 100 %, de préférence supérieure ou égale à 50 %, notamment allant de 50 % à 100 %.

En particulier, lorsque la composition comprend des deuxièmes particules solides primaires et/ou secondaires telles que décrites précédemment, ces particules sont présentes dans la composition en une teneur telle que la fraction volumique desdites premières particules solides et desdites deuxièmes particules solides primaires et/ou secondaires, est supérieure ou égale à 25,05 % (notamment allant de 25,05 % à 100 %) du volume total des premières et deuxièmes particules solides, avantageusement supérieure ou égale à 30,05 % (notamment allant de 30,05 % à 100 %), et de préférence supérieure ou égale à 40, 05 % (notamment allant de 40,05 % à 100 %), et mieux supérieure ou égale à 50,05 % (notamment allant de 50,05 % à 100 %).

La composition étant sous la forme d'une dispersion cire-dans-eau, elle comprend donc un mileu aqueux formant une phase continue aqueuse.

La composition peut comprendre également au moins une huile volatile et/ou un solvant organique volatile tels que ceux décrits ci après, qui sont dipsersés dans la phase aqueuse de la composition.

Dans la présente demande, on entend par « polymère apte à adhérer sur les matières kératiniques », appelé par la suite polymère adhérent, un polymère apte à rester fixé sur les matières kératiniques telles que les fibres kératiniques comme les cils , les cheveux, ou la peau, et de préférence sur les cils, lors du contact du polymère avec lesdites matières kératiniques. Un tel polymère adhérent a d'ailleurs une bonne aptitude à former un dépôt sur les matières kératiniques et reste fixé sur ces dernières.

Le polymère adhérent est un polymère filmogène à une température inférieure ou égale à 40 °C. Dans la présente demande, on entend par "polymère filmogène", un polymère apte à former à lui seul ou en présence d'un agent auxiliaire de filmification, un dépôt, notamment un film, continu et adhérent sur un support, notamment sur les matières kératiniques.

Le polymère adhérent présent dans la composition selon l'invention peut être un polymère solubilisé ou dispersé sous forme de particules solides dans une phase aqueuse de la composition ou bien encore solubilisé ou dispersé sous forme de particules solides dans une phase grasse liquide. La composition peut comprendre un mélange de ces polymères. Lorsque le polymère filmogène se présente sous forme de particules solides, ces particules peuvent présenter une taille moyenne de particules allant de 5 nm à 10 $\mu$m, notamment allant de 5 nm à 5 $\mu$m, avantageusement allant de 5 nm à 600 nm, et de préférence allant de 20 nm à 300 nm.

Le polymère adhérent peut être présent dans la composition selon l'invention en une teneur en matières sèches allant de 0,1 % à 50 % en poids par rapport au poids total de la composition, de préférence de 0,5 % à 40 % en poids, et mieux de 1 % à 30 % en poids.

Parmi les polymères adhérents utilisables dans la composition de la présente invention, on peut citer les polymères synthétiques, de type radicalaire ou de type polycondensat, les polymères d'origine naturelle, et leurs mélanges.

Par polymère radicalaire, on entend un polymère obtenu par polymérisation de monomères à insaturation notamment éthylénique, chaque monomère étant susceptible de s'homopolymériser (à l'inverse des polycondensats). Les polymères de type radicalaire peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyétherpolyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -$SO_3M$, avec M représentant un atome d'hydrogène, un ion ammonium $NH_4^+$ ou un ion alcalin, alcalino-terreux ou métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement -$SO_3M$.

Le noyau aromatique du monomère aromatique bifonctionnel portant en outre un groupement -$SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -$SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère adhérent peut être présent sous la forme de particules solides en dispersion aqueuse dans le milieu aqueux de la composition, connue généralement sous le nom de latex ou pseudolatex. Les techniques de préparation de ces dispersions sont bien connues de l'homme du métier.

Comme dispersion aqueuse de polymère adhérent filmogène, on peut utiliser les dispersions acryliques vendues sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane vendues sous les dénominations NEOREZ R-981®, NEOREZ R-974® par la société AVECIA-NEORESINS, les AVALURE UR-405®, AVALURE UR-410®, AVALURE UR-425®, AVALURE UR-450®, SANCURE 875®, SANCURE 861®, SANCURE 878®, SANCURE 2060® par la société GOODRICH, IMPRANIL 85® par la société BAYER, AQUAMERE H-1511® par la société HYDROMER.

Comme dispersion aqueuse de polymère adhérent, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère adhérent peut être un polymère hydrosoluble et est donc présent dans le milieu aqueux (la phase aqueuse) de la composition sous forme solubilisée. Comme exemples de polymères adhérents, notamment filmogènes, hydrosolubles, on peut citer

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique;
- les polymères d'origine naturelle, éventuellement modifiés, tels que :

  . les gommes arabiques, la gomme de guar, les dérivés du xanthane, la gomme de karaya ;
  . les alginates et les carraghénanes ;
  . les glycoaminoglycanes, l'acide hyaluronique et ses dérivés ;
  . la résine shellac, la gomme de sandaraque, les dammars, les élémis, les copals ;
  . l'acide désoxyribonucléïque ;
  . les muccopolysaccharides tels que l'acide hyaluronique, les chondroïtines sulfate, et leurs mélanges.

Selon une autre variante de réalisation de la composition selon l'invention, le polymère adhérent, notamment filmogène, peut être présent dans une phase grasse liquide dispersée dans la phase aqueuse (le mileu aqueux) de la composition, la phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit $10^5$ Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées ci-après.

Selon un troisième mode de réalisation de la composition selon l'invention, le polymère adhérent, notamment filmogène, peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère adhérent, notamment filmogène, peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une $\alpha$-oléfine (ayant de 8 à 28 atomes de carbone), un alkivinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui ont pour but qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyl éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère adhérent, notamment filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Selon un mode de réalisation préféré de la composition selon l'invention, le polymère adhérent peut être un polymère apte à former un dépôt, notamment un film, produisant à une concentration de 7 % dans l'eau, une rétraction du stratum corneum isolé de plus de 1 % à 30°C sous une humidité relative de 40 %, de préférence de plus de 1,2 %, et mieux de plus de 1,5 %. Cette rétraction est mesurée à l'aide d'un extensiomètre, selon la méthode décrite ci-après.

La composition selon l'invention peut comprendre en outre au moins une cire additionnelle différente de la cire dure décrite précédemment.

Comme cire additionnelle, on peut notamment citer la cire d'abeilles, la cire de lanoline, et les cires d'insectes de Chine; la cire de riz, la cire de Carnauba, , certaines cires microcristallines, les cires de paraffine, certaines ozokérites, certaines cires de polyéthylène, certaines cires obtenues par la synthèse de Fisher-Tropsch.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de tournesol hydrogénée, l'huile de ricin hydrogénée, l'huile de coprah hydrogénée et l'huile de lanoline hydrogénée. On peut encore citer les cires de silicone ou les cires fluorées.

Les cires additionnelles présentes dans la composition peuvent être dispersées sous forme de particules dans le milieu aqueux de la composition. Ces particules peuvent avoir une taille moyenne allant de 50 nm à 50 $\mu$m, et de préférence de 50 nm à 10 $\mu$m.

En particulier, les cires additionnelles sont généralement présentes dans la composition selon l'invention sous forme de particules solides et font donc parties des deuxièmes particules solides définies précédemment.

La cire additionnelle peut être présente dans la composition selon l'invention en une teneur allant de 0,1 % à 35 % en poids, par rapport au poids total de la composition, de préférence de 0,1 % à 20 % en poids, et mieux de 1 % à 10 % en poids.

La composition selon l'invention comprend un milieu aqueux, constituant une phase aqueuse, formant la phase continue de la composition.

Le milieu aqueux de la composition formant la phase aqueuse peut être constituée essentiellement d'eau ; elle peut également comprendre un mélange d'eau et de solvant miscible à l'eau (solvant apte à former avec l'eau un mélange homogène et transparent à l'oeil à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$.

La phase aqueuse (eau et éventuellement le solvant organique miscible à l'eau), ou milieu aqueux, peut être présente, en une teneur allant de 10 % à 95 % en poids, par rapport au poids total de la composition, de préférence de 20 % à 70 % en poids, et mieux de 30 % à 80 % en poids.

Selon un deuxième mode de réalisation de la composition selon l'invention, la composition peut comprendre au moins une huile ou solvant organique volatile dispersé qui peut notamment former une phase grasse dispersée dans la phase aqueuse.

Par " huile ou solvant organique volatile", on entend au sens de l'invention des solvants organiques et des huiles cosmétiques volatiles, liquides à température ambiante, ayant une pression de vapeur non nulle, à température ambiante et pression atmosphérique, allant en particulier de $10^{-2}$ à 300 mm de Hg (1,33 Pa à 40.000 Pa) et de préférence supérieure à 0,3 mm de Hg (40 Pa). Par "huile non volatile", on entend une huile ayant une pression de vapeur inférieure à $10^{-2}$ mm de Hg (1,33 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité $\leq$ 8 centistokes (8 $10^{-6}$ m$^2$/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile ou le solvant volatile peut être présent dans la composition selon l'invention en une teneur allant de 0,1 % à 30 % en poids, par rapport au poids total de la composition, de préférence de 0,1 % à 15 % en poids.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,

- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_5 + R_6$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;

et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0,1 % à 30 % en poids, et de préférence de 0,1 % à 15 % en poids, par rapport au poids total de la composition.

Avantageusement, lorsque la composition comprend une huile volatile et une huile non volatile, la teneur en huile volatile et en huile volatile dans la composition est inférieure ou égale à 49 % en poids, par rapport au poids total de la composition, de préférence inférieure à 25 % en poids, et mieux inférieure à 15 % en poids.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges.
- parmi les tensioactifs anioniques : les acides gras en $C_{16}$-$C_{30}$ neutralisés par les amines, l'ammoniaque ou les sels alcalins, et leurs mélanges ; le copolymère acide acrylique /itaconatee de monostéaryleoxyéthyléné (20 OE) en dispersion aqueuse à 30 % en poids vendu sous la dénomination « STRUCTURE 2001 » par la société National Starch, le copolymère acide acrylique/itaconate de monocétyle éthoxylé (20 OE) en dispersion aqueuse à 30 % vendu sous la dénomination « STRUCTURE 3001 » par la société National Starch.

On utilise de préférence des tensioactifs permettant l'obtention d'émulsion huile-dans-eau ou cire-dans-eau.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 50 % en poids, par rapport au poids total de la composition, de préférence allant de 0,01 % à 30 % en poids.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le $\beta$-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition de l'invention peut comprendre, en outre, tout additif usuellement utilisé en cosmétique tels que les antioxydants, les charges, les conservateurs, les parfums, les neutralisants, les épaississants, les actifs cosmétiques ou dermatologiques comme par exemple des émollients, des hydratants, des vitamines, des filtres solaires, et leurs mélanges. Ces additifs peuvent être présents dans la composition en une teneur allant de 0,01 à 20 % du poids total de la composition et mieux de 0,01 à 10% (si présents).

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique ou dermatologique.

**Méthode de mesure de la masse volumique de particules solides :**

La masse volumique apparente de particules solides est mesurée à l'aide d'un pycnomètre Gay-Lussac.

On utilise une balance de précision (précision de 1 mg) et les mesures sont effectuées dans une enceinte thermostatée à 25 °C ($\pm$ 0.5°C). On utilise également deux liquides de référence ayant une masse volumique Mv qui sont l'eau déminéralisée (MV = 1000 kg/m$^3$) et l'heptane (MV = 683.7 kg/m$^3$). On effectue la mesure de la masse volumique des particules solides avec chaque liquide de référence.

On place le pycnomètre et les produits utilisé pour effectuer la mesure à la température de 25°C. Les masses citées ci-après sont exprimées en kilogrammes.

On mesure la masse M0 du pycnomètre, puis on remplit complètement le pycnomètre du liquide de référence employé, en évitant l'introduction de bulle d'air.
On mesure la masse M1 du pycnomètre rempli.

Puis on prépare un mélange de masse M2 du matériau dont on veut mesurer sa masse volumique Mv2 avec une masse M3 de liquide de référence. On agite le mélange puis juste à la fin de l'agitation, on remplit le pycnomètre avec ce mélange et on mesure la masse M4 du pycnomètre rempli. On détermine ainsi la masse M4 - M0 du mélange présent dans le pycnomètre.
Le pycnomètre ayant un volume de remplissage constant, on peut donc établir la relation suivante : (M1-M0) / Mv= (M2/Mv2 + M3/Mv) x (M4-M0) / (M2+M3)

Cette relation permet de calculer la valeur de la masse volumique Mv2 des particules solides, exprimée en kg/m$^3$. On détermine ainsi pour chacun des liquides de référence une valeur de la masse volumique des particules solides. Selon l'invention, la valeur la plus élevée (parmi la masse volumique mesurée avec l'eau distillée et la masse volumique mesurée avec l'heptane) est retenue comme valeur de la masse volumique pour la détermination de la fraction volumique des particules solides.

**Méthode de mesure de rétraction d'un polymère :**

Le principe consiste à mesurer avant traitement et après traitement la longueur d'une éprouvette de stratum cornéum isolé et de déterminer le pourcentage de rétraction de l'éprouvette.

On utilise des éprouvettes de 1 cm X 0,4 cm de stratum cornéum d'épaisseur allant de 10 à 20 $\mu$m disposées sur l'extensiomètre MTT 610 commercialisé par la société DIASTRON.

L'éprouvette est placée entre 2 mâchoires puis laissée pendant 12 heures dans une atmosphère à 30 °C et 40 % d'humidité relative.

On tracte à la vitesse de 2 mm/minute l'éprouvette d'une longueur comprise entre 5 et 10 % de la longueur initiale pour déterminer la longueur $l_1$ à partir de laquelle l'éprouvette commence à exercer une force sur les mâchoires et détectée par l'appareil.

On détend ensuite l'éprouvette puis on applique sur le stratum cornéum 2 mg d'une composition aqueuse à 7 % en poids de polymère. Après évaporation totale de la composition, on tracte l'éprouvette dans les mêmes conditions que celles décrites précédemment pour déterminer également la longueur $l_2$ pour l'éprouvette traitée.

Le pourcentage de rétraction est déterminé par le rapport : 100 X $(l_2 - l_1)/l_1$.

L'invention est illustrée plus en détail dans les exemples suivants.

**Exemples 1 et 2 comparatifs :**

a) On a préparé 2 dispersions aqueuses de cire, l'une (dispersion 1) avec la cire vendue sous la dénomination « PHYTOWAX Olive 18 L 57 » par la société SOPHIM (cire de dureté égale à 10,05 Mpa), l'autre (dispersion 2) avec la cire d'abeille (cire de dureté égale à 3,68 MPa)

Chaque dispersion de cire est préparée en mélangeant à 95 °C, 40 g de cire, 4 g de tensioactif alcool laurylique polyoxyéthyléné à 23 motifs d'oxyde d'éthylène vendu sous la dénomination « BRIJ 35 » par la société UNICHEMA et 56 g d'eau chauffée à 95 °C, sous agitation à l'aide d'un agitateur Ultraturrax, jusqu'à obtenir une dispersion aqueuse de cire ayant une taille moyenne de particules d'environ 300 nm.

b) On a préparé 2 mascaras ayant la composition suivante :

- Cire 30 g
- Oxyde de fer noir (Sicovit noir 85E172 de BASF) 5 g
- Hydroxyéthylcellulose
  (Cellosize QP4400M d'Amerchol) 1 g
- Propylène glycol 5 g
- Tensioactif (Brij 35) 7,5 g
- Eau qsp 100 g

Le mascara selon l'invention (exemple 1) contient la cire vendue sous la dénomination « PHYTOWAX Olive 18 L 57 » par la société SOPHIM de la dispersion 1.
Le mascara ne faisant pas partie de l'invention (exemple 2) contient la cire d'abeille de la dispersion 2.
Chaque mascara est préparé en mélangeant 75 g de la dispersion de cire correspondante décrite au point a) précédent avec la fraction aqueuse complémentaire comprenant les autres ingrédients.
La fraction non volatile de ces 2 mascaras contient une fraction volumique de particules solides (cire, diamant, oxyde de fer noir) de 84 % (par rapport au volume total de la fraction non volatile).
Dans le mascara de l'exemple 1, la fraction volumique de la cire dure représente 96,8 % du volume total des particules solides. Le mascara de l'exemple 2 ne contient pas de cire dure.
On a mesuré les propriétés recourbantes de ces 2 mascaras selon le protocole suivant :
On a utilisé des éprouvettes de cheveux caucasiens comprenant 15 cheveux d'une longueur de 15 mm présentant un arc de courbure ayant un rayon de courbure compris entre environ 6 et 7 mm. Ces éprouvettes sont fixées sur un support de telle façon que le dessus de l'éprouvette correspond au côté interne de l'arc formé par l'éprouvette, le dessous de l'éprouvette correspondant au côté externe de l'arc formé par l'éprouvette..
Avant l'application du mascara, la courbure de l'éprouvette de cheveux a été mesurée en prenant une photo numérique de profil à l'aide du Macrozoom Navitar.
Puis on appliqué le mascara sur chaque éprouvette à l'aide d'une brosse sur le dessous de l'éprouvette. On a effectué 3 séries de 10 passages de la brosse avec une attente de 2 minutes entre chaque série de 10 passages.
20 minutes après le dernier passage de la brosse sur l'éprouvette, on a photographié l'éprouvette de cheveux maquillés.
Les images sont traitées avec le système d'analyse d'image Microvision et on détermine le rayon de courbure moyens des cheveux avant maquillage (Rc i) et le rayon de courbure moyen des cheveux après maquillage (Rcf), le rayon de courbure étant mesuré en millimètre.
Le recourbement R est calculé selon la formule :

$$R = 1/R_{ci} - 1/R_{cf}$$

Plus la valeur de R est grande, plus le recourbement des cils mesuré est important.
On a obtenu les résultats suivants :

Exemple 1 (invention) : R = 0,018 mm$^{-1}$

Exemple 2 (hors invention) : R = 0,012 mm$^{-1}$

On a ainsi constaté que les propriétés recourbantes du mascara de l'exemple 1 selon l'invention sont supérieures à celles du mascara de l'exemple 2. L'utilisation d'une cire plus dure présente dans le mascara de l'exemple 1 permet d'augmenter le recourbement des cils.

**Revendications**

1. Composition de revêtement des fibres kératiniques, notamment des cils, sous forme de dispersion cire-dans-eau comprenant, dans un milieu aqueux cosmétiquement acceptable, une fraction non volatile comprenant :

   - un polymère apte à adhérer sur les matières kératiniques qui est un polymère filmogène à une température inférieure ou égale à 40 °C,
   - des premières particules solides à 25 °C comprenant un premier matériau comprenant une cire ayant un point de fusion inférieur à 77 °C et une dureté supérieure ou égale à 6,5 MPa,
   - et éventuellement des deuxièmes particules solides comprenant un deuxième matériau différent du premier matériau, les deuxièmes particules solides n'étant pas aptes à coalescer à une température inférieure ou égale à 40 °C,
   les premières et, le cas échéant, les deuxièmes particules solides étant présentes dans la composition en une teneur telle que la fraction volumique des premières particules solides et, le cas échéant, des deuxièmes particules solides est supérieure ou égale à 70 % du volume total de la fraction non volatile de la composition, et, le cas échéant, la fraction volumique des premières particules solides est supérieure ou égale à 25 % du volume total des premières particules solides et des deuxièmes particules solides.

2. Composition selon la revendication 1, **caractérisée par le fait que** la cire à un point de fusion inférieur à 60°C.

3. Composition selon la revendication 1 ou 2, **caractérisée par le fait que** la cire a un point de fusion allant de 30 °C à 59 °C.

4. Composition selon l'une quelconque des revendications précédentes, **caractérisée par le fait que** la cire a un point de fusion allant de 35 °C à 59 °C.

5. Composition selon l'une quelconque des revendications précédentes, **caractérisée par le fait que** la cire a un point de fusion allant de 40°C à 50 °C,

6. Composition selon l'une quelconque des revendications précédentes, **caractérisée par le fait que** la cire a une dureté allant de 6,5 MPa à 20 MPa, notamment allant de 6,5 MPa à 15 Mpa, de préférence allant de 6,5 à 12 MPa.

7. Composition selon l'une quelconque des revendications précédentes, **caractérisée par le fait que** la cire a une dureté allant de 9,7 à 20 MPa, de préférence allant de 9,7 à 15 MPa, et mieux allant de de 9,7 à 12 à MPa.

8. Composition selon l'une quelconque des revendications 1 à 6, **caractérisée par le fait que** la cire est choisie parmi la cire de Candellila, la cire de jojoba hydrogénée, la cire de sumac, la cérésine, le stéarate d'octacosanyle, le stéarate de tétra-contanyle, la cire de Shellac, le fumarate de béhényle, le tétrastéarate de di-(triméthylol-1,1,1 propane), le tétrabéhénate de di-(triméthylol-1,1,1 propane), les ozokerites, la cire obtenue par hydrogénation d'huile d'olive estérifiée avec l'alcool stéarylique, les cires obtenues par hydrogénation d'huile de ricin estérifiée avec l'alcool cétylique.

9. Composition selon l'une quelconque des revendications précédentes, **caractérisée par le fait que** la cire est choisie parmi le tétrastéarate de di-(trimethylol-1,1,1 propane) et la cire d'olive obtenue par hydrogénation d'huile d'olive estérifiée avec l'alcool stéarylique.

10. Composition selon l'une quelconque des revendications précédentes, **caractérisée par le fait que** les premières particules solides ont une taille moyenne allant 50 nm à 50 $\mu$m, et de préférence de 100 nm à 10 $\mu$m.

11. Composition selon l'une quelconque des revendications précédentes, **caractérisée par le fait que** les premières particules de cire sont présentes dans la composition en une teneur allant de 1,25 % à 50 % en poids, par rapport au poids total de la composition, avantageusement allant de 5 % à 40 % en poids, et de préférence allant de 10 % à 25 % en poids.

**12.** Composition selon l'une quelconque des revendications précédentes, **caractérisée par le fait que** la composition comprend les premières et les deuxièmes particules solides.

**13.** Composition selon l'une quelconque des revendications précédentes, **caractérisée par le fait que** les deuxièmes particules solides sont choisies parmi :

- des deuxièmes particules solides primaires comprenant un matériau cristallin ou semi-cristallin solide à 25 °C présentant une transition de phase de premier ordre, de fusion ou de combustion, supérieure à 100°C ;
- et/ou des deuxièmes particules solides secondaires, comprenant un matériau amorphe ayant une température de transition vitreuse supérieure ou égale à 60 °C,
- et/ou d'autres particules solides à 25 °C, appelées deuxièmes particules solides tertiaires, différentes desdites deuxièmes particules primaires et secondaires
- et leurs mélanges.

**14.** Composition selon la revendication précédente, **caractérisée par le fait que le fait que** le matériau cristallin ou semi-cristallin présente une transition de phase de premier ordre supérieure à 120 °C.

**15.** Composition selon la revendication 13 ou 14, **caractérisée par le fait que** le matériau cristallin ou semi-cristallin présente une transition de phase de premier ordre supérieure à 150°C.

**16.** Composition selon l'une quelconque des revendications 13 à 15, **caractérisée par le fait que** le premier matériau a une dureté Vicker supérieure ou égale à 10, de préférence allant de 10 à 7500.

**17.** Composition selon l'une quelconque des revendications 13 à 16, **caractérisée par le fait que** le premier matériau a une dureté Vicker supérieure ou égale à 200, de préférence allant de 200 à 7500.

**18.** Composition selon l'une quelconque des revendications 13 à 17, **caractérisée par le fait que** le premier matériau a une dureté Vicker supérieure ou égale à 400, de préférence allant de 400 à 7500.

**19.** Composition selon l'une quelconque des revendications 13 à 18, **caractérisée par le fait que** ledit matériau est choisi dans le groupe formé par la silice, le verre, le diamant, le cuivre, le nitrure de bore, les céramiques, les micas, les oxydes métalliques, les polyamides, et leurs mélanges.

**20.** Composition selon la revendication 19, **caractérisée par le fait que** les oxydes métalliques sont choisis parmi les oxydes de fer.

**21.** Composition selon l'une quelconque des revendications 13 à 20, **caractérisée par le fait que** les deuxièmes particules primaires ont une taille moyenne allant de 50 nm à 50 $\mu$m, de préférence allant de 20 nm à 50 $\mu$m.

**22.** Composition selon la revendication 13, **caractérisée par le fait que** le matériau amorphe des deuxièmes particules solides secondaires a une température de transition vitreuse supérieure ou égale à 80°C

**23.** Composition selon l'une des revendications 13 ou 22, **caractérisée par le fait que** le deuxième matériau amorphe des deuxièmes particules solides secondaires a une température de transition vitreuse supérieure ou égale à 100 °C.

**24.** Composition selon l'une des revendications 13, 22 ou 23, **caractérisée par le fait que** le matériau amorphe est un polymère.

**25.** Composition selon l'une quelconque des revendications 13, 22 à 24, **caractérisée par le fait que** le matériau amorphe est un polymère choisi parmi les polyméres radicalaires et les polycondensats.

**26.** Composition selon l'une quelconque des revendications 13, 22 à 25, **caractérisée par le fait que** le matériau amorphe est un polymère choisi parmi les polymères d'éthylène, les polymères de propylène, les polymères acryliques, les polymères d'acrylamide, les polymères de (méth)acrylonitrile,les polycarbonates, les polyuréthanes, les polyesters, les polyamides, les polysulfones, les polysulfonamides, les carbohydrates.

**27.** Composition selon l'une quelconque des revendications 13 et 22 à 26, **caractérisée par le fait que** les deuxièmes particules solides secondaires ont une taille moyenne allant de 10 nm à 50 $\mu$m, et de préférence allant de 20 nm

à 1 μm.

**28.** Composition selon l'une quelconque des revendications précédentes, **caractérisée par le fait que** la fraction volumique des premières particules solides et, le cas échéant, des deuxièmes particules solides est supérieure ou égale à 70 %, notamment va de 70 % à 95 %, du volume total de la fraction non volatile de la composition.

**29.** Composition selon l'une quelconque des revendications précédentes, **caractérisée par le fait que** les premières particules sont présentes dans la composition en une teneur telle que la fraction volumique des premières particules solides est supérieure ou égale à 30 %, de préférence allant de 30 % à 100 %, de la fraction volumique totale des premières et deuxièmes particules solides.

**30.** Composition selon l'une quelconque des revendications précédentes, **caractérisée par le fait que** les premières particules sont présentes dans la composition en une teneur telle que la fraction volumique des premières particules solides est supérieure ou égale à 40 %, de préférence allant de 40 % à 100 %, de la fraction volumique totale des premières et deuxièmes particules solides.

**31.** Composition selon l'une quelconque des revendications précédentes, **caractérisée par le fait que** les premières particules sont présentes dans la composition en une teneur telle que la fraction volumique des premières particules solides est supérieure ou égale à 50 % de la fraction volumique totale des premières et deuxièmes particules solides, notamment allant de 50 % à 100 %.

**32.** Composition selon l'une quelconque des revendications précédentes, **caractérisée par le fait que** les deuxièmes particules solides primaires et/ou secondaires sont présentes dans la composition en une teneur telle que la fraction volumique desdites premières particules solides et desdites deuxièmes particules solides primaires et/ou secondaires, est supérieure ou égale à 25,05 %, de préférence allant de 25,05 % à 100 %, du volume total des premières et deuxièmes particules solides.

**33.** Composition selon l'une quelconque des revendications précédentes, **caractérisée par le fait que** les deuxièmes particules solides primaires et/ou secondaires sont présentes dans la composition en une teneur telle que la fraction volumique desdites premières particules solides et desdites deuxièmes particules solides primaires et/ou secondaires, est supérieure ou égale à 30,05 %, notamment allant de 30,05 % à 100 %, du volume total des premières et deuxièmes particules solides, et de préférence supérieure ou égale à 40, 05 %, notamment allant de 40,05 % à 100 %, et mieux supérieure ou égale à 50,05 %, notamment allant de 50,05 % à 100 %.

**34.** Composition selon l'une quelconque des revendications précédentes, **caractérisée par le fait que** le polymère apte à adhérer sur les matières kératiniques est choisi dans le groupe formé par les polymères vinyliques, les polyuréthanes, les polyesters, les polyamides, les polyurées, les polymères cellulosiques.

**35.** Composition selon l'une quelconque des revendications précédentes, **caractérisée par le fait que** le polymère apte à adhérer sur les matières kératiniques est un polymère apte à former un dépôt produisant à une concentration de 7 % dans l'eau, une rétraction du stratum corneum isolé de plus de 1 % à 30°C sous une humidité relative de 40 %.

**36.** Composition selon la revendication précédente **caractérisée par le fait que** la rétraction du stratum cornéum est de plus de 1,2 %, et mieux de plus de 1,5 %.

**37.** Composition selon l'une quelconque des revendications précédentes, **caractérisée par le fait que** le polymère apte à adhérer sur les matières kératiniques est solubilisé dans le milieu aqueux.

**38.** Composition selon l'une quelconque des revendications 1 à 36, **caractérisée par le fait que** le polymère apte à adhérer sur les matières kératiniques est sous forme de particules solides en dispersion aqueuse.

**39.** Composition selon l'une quelconque des revendications 1 à 36, **caractérisée par le fait que** le polymère apte à adhérer sur les matières kératiniques est solubilisé ou dispersé sous forme de particules stabilisées en surface dans une phase grasse liquide dispersée dans le milieu aqueux.

**40.** Composition selon l'une quelconque des revendications précédentes, **caractérisée par le fait que** polymère apte à adhérer sur les matières kératiniques est présent en une teneur allant de 0,1 % à 50 % en poids, par rapport au poids total de la composition, de préférence de 0,5 % à 40 % en poids, et mieux de 1 % à 30 % en poids.

**41.** Composition selon l'une quelconque des revendications précédentes, **caractérisée par le fait qu'**elle comprend une cire additionnelle différente de la cire des premières particules solides.

**42.** Composition selon la revendication précédente , **caractérisée par le fait que** la cire additionnelle est présente dans la composition en une teneur allant de 0,1 % à 35 % en poids, par rapport au poids total de la composition, de préférence de 0,1 % à 20 % en poids, et mieux de 1 % à 10 % en poids.

**43.** Composition selon l'une la revendication 41 ou 42, **caractérisée par le fait que** la cire additionnelle est sous forme de particules ayant une taille moyenne allant de 50 nm à 50 $\mu$m, et de préférence de 50 nm à 10 $\mu$m.

**44.** Composition selon l'une quelconque des revendications précédentes, **caractérisée par le fait que** le milieu aqueux est formé d'eau ou d'un mélange d'eau et de solvant organique miscible à l'eau.

**45.** Composition selon la revendication précédente, **caractérisée par le fait que** le solvant organique miscible à l'eau est choisi parmi les monoalcools inférieurs ayant de 1 à 5 atomes de carbone, les glycols ayant de 2 à 8 atomes de carbone, les cétones en $C_3$-$C_4$, les aldéhydes en $C_2$-$C_4$.

**46.** Composition selon l'une quelconque des revendications précédentes, **caractérisée par le fait que** le milieu aqueux est présent en une teneur allant de 10 % à 95 % en poids, par rapport au poids total de la composition, de préférence de 20 % à 70 % en poids, et mieux de 30 % à 80 % en poids.

**47.** Composition selon l'une quelconque des revendications précédentes, **caractérisée par le fait qu'**elle comprend une huile volatile ou un solvant organique volatile.

**48.** Composition selon la revendication précédente, **caractérisée par le fait que** l'huile volatile est choisies parmi les huiles hydrocarbonées, les huiles siliconées, les huiles fluorées, ou leurs mélanges.

**49.** Composition selon l'une revendications 47 ou 48, **caractérisée par le fait que** l'huile volatile est présente en une teneur allant de 0,1 % à 30% en poids, par rapport au poids total de la composition, de préférence allant de 0,1 % à 15 % en poids.

**50.** Composition selon l'une quelconque des revendications précédentes, **caractérisée par le fait qu'**elle comprend une huile non volatile.

**51.** Composition selon la revendication précédente, **caractérisée par le fait que** l'huile non volatile est présente en une teneur allant de 0,1 % à 50 % en poids, de préférence de 0,1 % à 30 % en poids, par rapport au poids total de la composition.

**52.** Composition selon l'une quelconque des revendications précédentes, **caractérisée par le fait qu'**elle comprend un tensioactif.

**53.** Composition selon l'une quelconque des revendications précédentes, **caractérisée par le fait qu'** elle comprend un additif choisi parmi les matières colorantes, les antioxydants, les charges, les conservateurs, les parfums, les neutralisants, les épaississants, les actifs cosmétiques, les filtres solaires, les agents de coalescence, les plastifiants.

**54.** Composition selon l'une quelconque des revendications précédentes, **caractérisée par le fait qu'**elle est un mascara.

**55.** Procédé de maquillage ou de soin non thérapeutique des fibres kératiniques, notamment des cils, comprenant l'application sur les fibres kératiniques d'une composition selon l'une quelconque des revendications 1 à 54.

**56.** Utilisation d'une composition de revêtement des fibres kératiniques selon l'une quelconque des revendications 1 à 54, pour recourber les fibres kératiniques, notamment les cils.

**Claims**

**1.** Composition for coating keratin fibres, especially the eyelashes, in the form of a wax-in-water dispersion comprising, in a cosmetically acceptable aqueous medium, a non-volatile fraction comprising:

- a polymer that is capable of adhering to keratin materials, this polymer being film-forming at a temperature of less than or equal to 40°C,
- first particles that are solid at 25°C, comprising a first material comprising a wax with a melting point of less than 77°C and a hardness of greater than or equal to 6.5 MPa, and
- optionally second solid particles comprising a second material different from the first material, the second solid particles not being capable of coalescing at a temperature of less than or equal to 40°C,

the first and, where appropriate, second solid particles being present in the composition in a content such that the volume fraction of the first solid particles and, where appropriate, of the second solid particles is greater than or equal to 70% of the total volume of the non-volatile fraction of the composition, and

where appropriate, the volume fraction of the first solid particles is greater than or equal to 25% of the total volume of the first solid particles and of the second solid particles.

2. Composition according to Claim 1, **characterized in that** the wax has a melting point of less than 60°C.

3. Composition according to Claim 1 or 2, **characterized in that** the wax has a melting point ranging from 30°C to 59°C.

4. Composition according to any one of the preceding claims, **characterized in that** the wax has a melting point ranging from 35°C to 59°C.

5. Composition according to any one of the preceding claims, **characterized in that** the wax has a melting point ranging from 40°C to 50°C.

6. Composition according to any one of the preceding claims, **characterized in that** the wax has a hardness ranging from 6.5 MPa to 20 MPa, especially ranging from 6.5 MPa to 15 MPa and preferably ranging from 6.5 MPa to 12 MPa.

7. Composition according to any one of the preceding claims, **characterized in that** the wax has a hardness ranging from 9.7 to 20 MPa, preferably ranging from 9.7 to 15 MPa and better still ranging from 9.7 to 12 MPa.

8. Composition according to any one of Claims 1 to 6, **characterized in that** the wax is chosen from candelilla wax, hydrogenated jojoba wax, sumach wax, ceresin, octacosanyl stearate, tetracontanyl stearate, shellac wax, behenyl fumarate, bis(1,1,1-trimethylolpropane) tetrastearate, bis(1,1,1-trimethylolpropane) tetra-behenate, ozokerites, the wax obtained by hydrogenation of olive oil esterified with stearyl alcohol, and the waxes obtained by hydrogenation of castor oil esterified with cetyl alcohol.

9. Composition according to any one of the preceding claims, **characterized in that** the wax is chosen from bis(1,1,1-trimethylolpropane) tetrastearate and the olive wax obtained by hydrogenation of olive oil esterified with stearyl alcohol.

10. Composition according to any one of the preceding claims, **characterized in that** the first solid particles have a mean size ranging from 50 nm to 50 $\mu$m and preferably from 100 nm to 10 $\mu$m.

11. Composition according to any one of the preceding claims, **characterized in that** the first wax particles are present in the composition in a content ranging from 1.25% to 50% by weight, advantageously ranging from 5% to 40% by weight and preferably ranging from 10% to 25% by weight relative to the total weight of the composition.

12. Composition according to any one of the preceding claims, **characterized in that** the composition comprises the first and second solid particles.

13. Composition according to any one of the preceding claims, **characterized in that** the second solid particles are chosen from:

- primary second solid particles comprising a crystalline or semicrystalline material that is solid at 25°C, with a first-order phase transition, of melting or of combustion, of greater than 100°C; and/or
- secondary second solid particle comprising an amorphous material with a glass transition temperature of greater than or equal to 60°C, and/or
- other particles that are solid at 25°C, known as tertiary second solid particles, which are different from the said primary and secondary second particles, and
- mixtures thereof.

14. Composition according to the preceding claim, **characterized in that** the crystalline or semicrystalline material has a first-order phase transition of greater than 120°C.

15. Composition according to Claim 13 or 14, **characterized in that** the crystalline or semicrystalline material has a first-order phase transition of greater than 150°C.

16. Composition according to any one of Claims 13 to 15, **characterized in that** the first material has a Vickers hardness of greater than or equal to 10, preferably ranging from 10 to 7500.

17. Composition according to any one of Claims 13 to 16, **characterized in that** the first material has a Vickers hardness of greater than or equal to 200, preferably ranging from 200 to 7500.

18. Composition according to any one of Claims 13 to 17, **characterized in that** the first material has a Vickers hardness of greater than or equal to 400, preferably ranging from 400 to 7500.

19. Composition according to any one of Claims 13 to 18, **characterized in that** the said material is chosen from the group formed by silica, glass, diamond, copper, boron nitride, ceramics, micas, metal oxides and polyamides, and mixtures thereof.

20. Composition according to Claim 19, **characterized in that** the metal oxides are chosen from iron oxides.

21. Composition according to any one of Claims 13 to 20, **characterized in that** the primary second particles have a mean size ranging from 50 nm to 50 $\mu$m and preferably ranging from 20 nm to 50 $\mu$m.

22. Composition according to Claim 13, **characterized in that** the amorphous material of the secondary second solid particles has a glass transition temperature of greater than or equal to 80°C.

23. Composition according to either of Claims 13 and 22, **characterized in that** the second amorphous material of the secondary second solid particles has a glass transition temperature of greater than or equal to 100°C.

24. Composition according to one of Claims 13, 22 and 23, **characterized in that** the amorphous material is a polymer.

25. Composition according to any one of Claims 13 and 22 to 24, **characterized in that** the amorphous material is a polymer chosen from radical polymers and polycondensates.

26. Composition according to any one of Claims 13 and 22 to 25, **characterized in that** the amorphous material is a polymer chosen from ethylene polymers, propylene polymers, acrylic polymers, acrylamide polymers, (meth)acrylonitrile polymers, polycarbonates, polyurethanes, polyesters, polyamides, polysulfones, polysulfonamides and carbohydrates.

27. Composition according to any one of Claims 13 and 22 to 26, **characterized in that** the secondary second solid particles have a mean size ranging from 10 nm to 50 $\mu$m and preferably ranging from 20 nm to 1 $\mu$m.

28. Composition according to any one of the preceding claims, **characterized in that** the volume fraction of the first solid particles and, where appropriate, of the second solid particles is greater than or equal to 70%, especially ranging from 70% to 95%, of the total volume of the non-volatile fraction of the composition.

29. Composition according to any one of the preceding claims, **characterized in that** the first particles are present in the composition in a content such that the volume fraction of the first solid particles is greater than or equal to 30%, preferably ranging from 30% to 100%, of the total volume fraction of the first and second solid particles.

30. Composition according to any one of the preceding claims, **characterized in that** the first particles are present in the composition in a content such that the volume fraction of the first solid particles is greater than or equal to 40%, preferably ranging from 40% to 100%, of the total volume fraction of the first and second solid particles.

31. Composition according to any one of the preceding claims, **characterized in that** the first particles are present in the composition in a content such that the volume fraction of the first solid particles is greater than or equal to 50%, especially ranging from 50% to 100%, of the total volume fraction of the first and second solid particles.

32. Composition according to any one of the preceding claims, **characterized in that** the primary and/or secondary second solid particles are present in the composition in a content such that the volume fraction of the said first solid particles and of the said primary and/or secondary second solid particles is greater than or equal to 25.05%, preferably ranging from 25.05% to 100%, of the total volume of the first and second solid particles.

33. Composition according to any one of the preceding claims, **characterized in that** the primary and/or secondary second solid particles are present in the composition in a content such that the volume fraction of the said first solid particles and of the said primary and/or secondary second solid particles is greater than or equal to 30.05%, especially ranging from 30.05% to 100%, preferably greater than or equal to 40.05%, especially ranging from 40.05% to 100%, and better still greater than or equal to 50.05%, especially ranging from 50.05% to 100%, of the total volume of the first and second solid particles.

34. Composition according to any one of the preceding claims, **characterized in that** the polymer that is capable of adhering to keratin materials is chosen from the group formed by vinyl polymers, polyurethanes, polyesters, polyamides, polyureas and cellulose polymers.

35. Composition according to any one of the preceding claims, **characterized in that** the polymer that is capable of adhering to keratin materials is a polymer that is capable of forming a deposit that produces, at a concentration of 7% in water, shrinkage of isolated stratum corneum of more than 1% at 30°C under a relative humidity of 40%.

36. Composition according to the preceding claim, **characterized in that** the shrinkage of the stratum corneum is more than 1.2% and better still more than 1.5%.

37. Composition according to any one of the preceding claims, **characterized in that** the polymer that is capable of adhering to keratin materials is dissolved in the aqueous medium.

38. Composition according to any one of Claims 1 to 36, **characterized in that** the polymer that is capable of adhering to keratin materials is in the form of solid particles in aqueous dispersion.

39. Composition according to any one of Claims 1 to 36, **characterized in that** the polymer that is capable of adhering to keratin materials is dissolved or dispersed in the form of surface-stabilized particles in a liquid fatty phase dispersed in the aqueous medium.

40. Composition according to any one of the preceding claims, **characterized in that** the polymer that is capable of adhering to keratin materials is present in a content ranging from 0.1% to 50% by weight, preferably from 0.5% to 40% by weight and better still from 1% to 30% by weight relative to the total weight of the composition.

41. Composition according to any one of the preceding claims, **characterized in that** it comprises an additional wax other than the wax of the first solid particles.

42. Composition according to the preceding claim, **characterized in that** the additional wax is present in the composition in a content ranging from 0.1% to 35% by weight, preferably from 0.1% to 20% by weight and better still from 1% to 10% by weight relative to the total weight of the composition.

43. Composition according to either of Claims 41 and 42, **characterized in that** the additional wax is in the form of particles with a mean size ranging from 50 nm to 50 $\mu$m and preferably from 50 nm to 10 $\mu$m.

44. Composition according to any one of the preceding claims, **characterized in that** the aqueous medium is formed from water or from a mixture of water and of water-miscible organic solvent.

45. Composition according to the preceding claim, **characterized in that** the water-miscible organic solvent is chosen from lower monoalcohols containing from 1 to 5 carbon atoms, glycols containing from 2 to 8 carbon atoms, $C_3$-$C_4$ ketones and $C_2$-$C_4$ aldehydes.

**46.** Composition according to any one of the preceding claims, **characterized in that** the aqueous medium is present in a content ranging from 10% to 95% by weight, preferably from 20% to 70% by weight and better still from 30% to 80% by weight relative to the total weight of the composition.

**47.** Composition according to any one of the preceding claims, **characterized in that** it comprises a volatile oil or a volatile organic solvent.

**48.** Composition according to the preceding claim, **characterized in that** the volatile oil is chosen from hydrocarbon-based oils, silicone oils and fluoro oils, or mixtures thereof.

**49.** Composition according to either of Claims 47 and 48, **characterized in that** the volatile oil is present in a content ranging from 0.1% to 30% by weight and preferably ranging from 0.1% to 15% by weight relative to the total weight of the composition.

**50.** Composition according to any one of the preceding claims, **characterized in that** it comprises a non-volatile oil.

**51.** Composition according to the preceding claim, **characterized in that** the non-volatile oil is present in a content ranging from 0.1% to 50% by weight and preferably from 0.1% to 30% by weight relative to the total weight of the composition.

**52.** Composition according to any one of the preceding claims, **characterized in that** it comprises a surfactant.

**53.** Composition according to any one of the preceding claims, **characterized in that** it comprises an additive chosen from dyestuffs, antioxidants, fillers, preserving agents, fragrances, neutralizers, thickeners, cosmetic active agents, sunscreens, coalescers and plasticizers.

**54.** Composition according to any one of the preceding claims, **characterized in that** it is a mascara.

**55.** Non-therapeutic process for making up or caring for keratin fibres, especially the eyelashes, comprising the application to the keratin fibres of a composition according to any one of Claims 1 to 54.

**56.** Use of a composition for coating keratin fibres according to any one of Claims 1 to 54, for curling the keratin fibres, especially the eyelashes.

**Patentansprüche**

**1.** Zusammensetzung zum Überziehen von Keratinfasern, insbesondere von Wimpern, in Form einer Wachs-in-Wasser-Dispersion, die in einem kosmetisch akzeptablen wässrigen Medium einen nichtflüchtigen Anteil enthält, mit:

einem Polymer, das an den Keratinsubstanzen haften kann und bei dem es sich um ein Polymer handelt, das bei einer Temperatur von 40 °C oder darunter filmbildend ist,
ersten, bei 25 °C festen Partikeln, die ein erstes Material umfassen, das ein Wachs mit einem Schmelzpunkt unter 77°C und einer Härte größer oder gleich 6,5 MPa enthält, und
- gegebenenfalls zweiten festen Partikeln, die ein zweites Material umfassen, das von dem ersten Material verschieden ist, wobei die zweiten festen Partikel bei einer Temperatur von 40 °C oder darunter nicht zur Koaleszenz befähigt sind,
- wobei die ersten festen Partikel und gegebenenfalls die zweiten festen Partikel in der Zusammensetzung in einer solchen Menge enthalten sind, dass der Volumenanteil der ersten festen Partikel und gegebenenfalls der zweiten festen Partikel 70 % oder mehr als 70 % des Gesamtvolumens des nichtflüchtigen Anteils der Zusammensetzung beträgt,
und wobei gegebenenfalls der Volumenanteil der ersten festen Partikel größer oder gleich 25 % des Gesamtvolumens der ersten und zweiten festen Partikel ist.

**2.** Zusammensetzung nach Anspruch 1, **dadurch gekennzeichnet, dass** das Wachs einen Schmelzpunkt unter 60 °C aufweist.

**3.** Zusammensetzung nach Anspruch 1 oder 2, **dadurch gekennzeichnet, dass** das Wachs einen Schmelzpunkt im Bereich von 30 bis 59 °C besitzt.

4. Zusammensetzung nach einem der vorhergehenden Ansprüche, **dadurch gekennzeichnet, dass** das Wachs einen Schmelzpunkt im Bereich von 35 bis 59 °C besitzt.

5. Zusammensetzung nach einem der vorhergehenden Ansprüche, **dadurch gekennzeichnet, dass** das Wachs einen Schmelzpunkt im Bereich von 40 bis 50 °C besitzt.

6. Zusammensetzung nach einem der vorhergehenden Ansprüche, **dadurch gekennzeichnet, dass** das Wachs eine Härte von 6,5 bis 20 MPa, insbesondere von 6, 5 bis 15 MPa und vorzugsweise von 6,5 bis 12 MPa aufweist.

7. Zusammensetzung nach einem der vorhergehenden Ansprüche, **dadurch gekennzeichnet, dass** das Wachs eine Härte von 9,7 bis 20 MPa, vorzugsweise von 9,7 bis 15 MPa und besser von 9,7 bis 12 MPa aufweist.

8. Zusammensetzung nach einem der Ansprüche 1 bis 6, **dadurch gekennzeichnet, dass** das Wachs unter Candelillawachs, hydriertem Jojobawachs, Sumachwachs, Ceresin, Octacosanylstearat, Tetracontanylstearat, Schellack, Behenylfumarat, Di(1,1,1-trimethylol-propan)-tetrastearat, Di(1,1,1-trimethylol-propan)-tetrabehenat, Ozokeriten, Wachs, das durch Hydrierung von mit Stearylalkohol verestertem Olivenöl erhalten wird, und Wachsen, das durch Hydrierung von mit Cetylalkohol verestertem Ricinusöl erhalten werden, ausgewählt ist.

9. Zusammensetzung nach einem der Ansprüche 1 bis 6, **dadurch gekennzeichnet, dass** das Wachs unter Di(1,1,1-trimethylolpropan)-tetrastearat und Olivenwachs, das durch Hydrierung von mit Stearylalkohol verestertem Olivenöl erhalten wird, ausgewählt ist.

10. Zusammensetzung nach einem der vorhergehenden Ansprüche, **dadurch gekennzeichnet, dass** die ersten festen Partikel eine mittlere Größe von 50 nm bis 50 $\mu$m und vorzugsweise 100 nm bis 10 $\mu$m aufweisen.

11. Zusammensetzung nach einem der vorhergehenden Ansprüche, **dadurch gekennzeichnet, dass** die ersten Partikel aus Wachs in einem Mengenanteil im Bereich von 1,25 bis 50 Gew.-%, bezogen auf das Gesamtgewicht der Zusammensetzung, vorteilhaft im Bereich von 5 bis 40 Gew.-% und vorzugsweise im Bereich von 10 bis 25 Gew.-% in der Zusammensetzung enthalten sind.

12. Zusammensetzung nach einem der vorhergehenden Ansprüche, **dadurch gekennzeichnet, dass** die Zusammensetzung erste feste Partikel und zweite feste Partikel enthält.

13. Zusammensetzung nach einem der vorhergehenden Ansprüche, **dadurch gekennzeichnet, dass** die zweiten festen Partikel ausgewählt sind unter:

- primären zweiten festen Partikeln, die ein kristallines oder - teilkristallines, bei 25 °C festes Material umfassen, das eine Phasenübergangstemperatur erster Ordnung, Schmelztemperatur oder Verdampfungstemperatur, über 100 °C aufweist,
- und/oder sekundären zweiten festen Partikeln, die ein amorphes Material mit einer Glasübergangstemperatur von größer oder gleich 60 °C umfassen,
- und/oder anderen bei 25 °C festen Partikeln, die als tertiäre zweite feste Partikel bezeichnet werden und die von den primären und sekundären zweiten Partikeln verschieden sind,
- und deren Gemischen.

14. Zusammensetzung nach dem vorhergehenden Anspruch, **dadurch gekennzeichnet, dass** das kristalline oder teilkristalline Material einen Phasenübergang erster Ordnung über 120 °C besitzt.

15. Zusammensetzung nach Anspruch 13 oder 14, **dadurch gekennzeichnet, dass** das kristalline oder teilkristalline Material einen Phasenübergang erster Ordnung über 150 °C besitzt.

16. Zusammensetzung nach einem der Ansprüche 13 bis 15, **dadurch gekennzeichnet, dass** das erste Material eine Vickers-Härte von größer oder gleich 10 und vorzugsweise 10 bis 7500 besitzt.

17. Zusammensetzung nach einem der Ansprüche 13 bis 16, **dadurch gekennzeichnet, dass** das erste Material eine Vickers-Härte von größer oder gleich 200 und vorzugsweise 200 bis 7500 besitzt.

18. Zusammensetzung nach einem der Ansprüche 13 bis 17, **dadurch gekennzeichnet, dass** das erste Material eine Vickers-Härte von größer oder gleich 400 und vorzugsweise 400 bis 7500 besitzt.

19. Zusammensetzung nach einem der Ansprüche 13 bis 18, **dadurch gekennzeichnet, dass** das Material unter Siliciumdioxid, Glas, Diamant, Kupfer, Bornitrid, Keramiken, Glimmern, Metalloxiden, Polyamiden und deren Gemischen ausgewählt ist.

20. Zusammensetzung nach Anspruch 19, **dadurch gekennzeichnet, dass** die Metalloxide unter den Eisenoxiden ausgewählt sind.

21. Zusammensetzung nach einem der Ansprüche 13 bis 20, **dadurch gekennzeichnet, dass** die primären zweiten Partikel eine mittlere Größe von 50 nm bis 50 $\mu$m und vorzugsweise 20 nm bis 50 $\mu$m aufweisen.

22. Zusammensetzung nach Anspruch 13, **dadurch gekennzeichnet, dass** das amorphe Material der sekundären zweiten festen Partikel eine Glasübergangstemperatur größer oder gleich 80 °C aufweist.

23. Zusammensetzung nach einem der Ansprüche 13 oder 22, **dadurch gekennzeichnet, dass** das amorphe Material der sekundären zweiten festen Partikel eine Glasübergangstemperatur größer oder gleich 100 °C aufweist.

24. Zusammensetzung nach einem der Ansprüche 13, 22 oder 23, **dadurch gekennzeichnet, dass** es sich bei dem amorphen Material um ein Polymer handelt.

25. Zusammensetzung nach einem der Ansprüche 13, 22 bis 24, **dadurch gekennzeichnet, dass** das amorphe Material ein Polymer ist, das unter den durch radikalische Polymerisation hergestellten Polymeren und den Polykondensaten ausgewählt ist.

26. Zusammensetzung nach einem der Ansprüche 13, 22 bis 25, **dadurch gekennzeichnet, dass** das amorphe Material ein Polymer ist, das unter den Polymeren von Ethylen, Polymeren von Propylen, Acrylpolymeren, Acrylamidpolymeren, (Meth)acrylnitrilpolymeren, Polycarbonaten, Polyurethanen, Polyestern, Polyamiden, Polysulfonen, Polysulfonamiden und Kohlenhydraten ausgewählt ist.

27. Zusammensetzung nach einem der Ansprüche 13 und 22 bis 26, **dadurch gekennzeichnet, dass** die sekundären zweiten festen Partikel eine mittlere Größe von 10 nm bis 50 $\mu$m und vorzugsweise 20 nm bis 1 $\mu$m besitzen.

28. Zusammensetzung nach einem der vorhergehenden Ansprüche, **dadurch gekennzeichnet, dass** der Volumenanteil der ersten festen Partikel und gegebenenfalls der zweiten festen Partikel mindestens 70 % und insbesondere 70 bis 95 % des Gesamtvolumens des nichtflüchtigen Anteils der Zusammensetzung beträgt.

29. Zusammensetzung nach einem der vorhergehenden Ansprüche, **dadurch gekennzeichnet, dass** die ersten Partikel in der Zusammensetzung in einem solchen Mengenanteil enthalten sind, dass der Volumenanteil der ersten festen Partikel mindestens 30 % und vorzugsweise 30 bis 100 % des Gesamtvolumenanteils der ersten und zweiten festen Partikel ausmacht.

30. Zusammensetzung nach einem der vorhergehenden Ansprüche, **dadurch gekennzeichnet, dass** die ersten Partikel in der Zusammensetzung in einem solchen Mengenanteil enthalten sind, dass der Volumenanteil der ersten festen Partikel mindestens 40 % und vorzugsweise 40 bis 100 % des Gesamtvolumenanteils der ersten und zweiten festen Partikel ausmacht.

31. Zusammensetzung nach einem der vorhergehenden Ansprüche, **dadurch gekennzeichnet, dass** die ersten Partikel in der Zusammensetzung in einem solchen Mengenanteil enthalten sind, dass der Volumenanteil der ersten festen Partikel mindestens 50 % und vorzugsweise 50 bis 100 % des Gesamtvolumenanteils der ersten und zweiten festen Partikel ausmacht.

32. Zusammensetzung nach einem der vorhergehenden Ansprüche; **dadurch gekennzeichnet, dass** die primären und/oder sekundären zweiten festen Partikel in der Zusammensetzung in einem solchen Mengenanteil enthalten sind, dass der Volumenanteil der ersten festen Partikel und der primären und/oder sekundären zweiten festen partikel größer oder gleich 25,05 % ist und vorzugsweise 25,05 bis 100 % des Gesamtvolumens der ersten und zweiten festen Partikel ausmacht.

**33.** Zusammensetzung nach einem der Ansprüche, **dadurch gekennzeichnet, dass** die primären und/oder sekundären zweiten festen Partikel in der Zusammensetzung in einem solchen Mengenanteil enthalten sind, dass der Volumenanteil der ersten festen Partikel und der primären und/oder sekundären zweiten festen Partikel mindestens 30,05 %, insbesondere 30,05 bis 100 % des Gesamtvolumens der ersten und zweiten festen Partikel und vorzugsweise mindestens 40,05 %, insbesondere 40,05 bis 100 % und noch besser mindestens 50,05 % und besonders 50,05 bis 100 % ausmacht.

**34.** Zusammensetzung nach einem der vorhergehenden Ansprüche, **dadurch gekennzeichnet, dass** das Polymer, das befähigt ist, an den Keratinsubstanzen zu haften, unter den Vinylpolymeren, Polyurethanen, Polyestern, Polyamiden, Polyharnstoffen und Cellulosepolymeren ausgewählt ist.

**35.** Zusammensetzung nach einem der vorhergehenden Ansprüche, **dadurch gekennzeichnet, dass** das Polymer, das befähigt ist, an den Keratinsubstanzen zu haften, ein Polymer ist, das eine Abscheidung bilden kann, die in einer Konzentration von 7 % in Wasser bei 30 °C und einer relativen Feuchte von 40 % eine Retraktion von isoliertem Stratum corneum von mehr als 1 % hervorruft.

**36.** Zusammensetzung nach dem vorhergehenden Anspruch, **dadurch gekennzeichnet, dass** die Retraktion des Stratum corneums mehr als 1,2 % und besser mehr als 1,5 % beträgt,

**37.** Zusammensetzung nach einem der vorhergehenden Ansprüche, **dadurch gekennzeichnet, dass** das Polymer, das befähigt ist, an den Keratinsubstanzen zu haften, in dem wässrigen Medium solubilisiert ist.

**38.** Zusammensetzung nach einem der Ansprüche 1 bis 36, **dadurch gekennzeichnet, dass** das Polymer, das befähigt ist, an den Keratinsubstanzen zu haften, in Form von festen Partikeln in wässriger Dispersion vorliegt.

**39.** Zusammensetzung nach einem der Ansprüche 1 bis 36, **dadurch gekennzeichnet, dass** das Polymer, das befähigt ist, an den Keratinsubstanzen zu haften, in Form von an der Oberfläche stabilisierten Partikeln in einer flüssigen, in der wässrigen Phase dispergierten Fettphase gelöst oder dispergiert ist.

**40.** Zusammensetzung nach einem der vorhergehenden Ansprüche, **dadurch gekennzeichnet, dass** das Polymer, das befähigt ist, an den Keratinsubstanzen zu haften, in einem Mengenanteil von 0,1 bis 50 Gew.-%, bezogen auf das Gesamtgewicht der Zusammensetzung, vorzugsweise 0,5 bis 40 Gew.-% und besser 1 bis 30 Gew.-% enthalten ist.

**41.** Zusammensetzung nach einem der vorhergehenden Ansprüche, **dadurch gekennzeichnet, dass** sie ein ergänzendes Wachs enthält, das von dem Wachs der ersten festen Partikeln verschieden ist.

**42.** Zusammensetzung nach dem vorhergehenden Anspruch, **dadurch gekennzeichnet, dass** das ergänzende Wachs in der Zusammensetzung in einem Mengenanteil von 0,1 bis 35 Gew.-%, bezogen auf das Gesamtgewicht der Zusammensetzung, vorzugsweise 0,1 bis 20 Gew.-% und besser 1 bis 10 Gew, -% enthalten ist.

**43.** Zusammensetzung nach einem der Ansprüche 41 oder 42, **dadurch gekennzeichnet, dass** das ergänzende Wachs in Form von Partikeln mit einer mittleren Größe von 50 nm bis 50 $\mu$m und vorzugsweise 50 nm bis 10 $\mu$m vorliegt.

**44.** Zusammensetzung nach einem der vorhergehenden Ansprüche, **dadurch gekennzeichnet, dass** das wässrige Medium aus Wasser oder einem Gemisch von Wasser und einem organischen, mit Wasser mischbaren Lösungsmittel gebildet ist.

**45.** Zusammensetzung nach dem vorhergehenden Anspruch, **dadurch gekennzeichnet, dass** das organische, mit Wasser mischbare Lösungsmittel unter den niederen Monoalkoholen mit 1 bis 5 Kohlenstoffatomen, Glycolen mit 2 bis 8 Kohlenstoffatomen, $C_{3-4}$-Ketonen und $C_{2-4}$-Aldehyden ausgewählt ist.

**46.** Zusammensetzung nach einem der vorhergehenden Ansprüche, **dadurch gekennzeichnet, dass** das wässrige Medium in einer Menge von 10 bis 95 Gew.-%, bezogen auf das Gesamtgewicht der Zusammensetzung, vorzugsweise 20 bis 70 Gew.-% und besser 30 bis 80 Gew.-% enthalten ist.

**47.** Zusammensetzung nach einem der vorhergehenden Ansprüche, **dadurch gekennzeichnet, dass** sie ein flüchtiges Öl oder ein flüchtiges organisches Lösemittel enthält.

48. Zusammensetzung nach dem vorhergehenden Anspruch, **dadurch gekennzeichnet, dass** das flüchtige Öl unter den Kohlenwasserstoffölen, Siliconölen, fluorierten Ölen oder deren Gemischen ausgewählt ist.

49. Zusammensetzung nach einem der Ansprüche 47 oder 48, **dadurch gekennzeichnet, dass** das flüchtige Öl in einer Menge von 0,1 bis 30 Gew.-%, bezogen auf das Gesamtgewicht der Zusammensetzung, und vorzugsweise 0,1 bis 15 Gew.-% enthalten ist.

50. Zusammensetzung nach einem der vorhergehenden Ansprüche, **dadurch gekennzeichnet, dass** sie ein nichtflüchtiges Öl enthält.

51. Zusammensetzung nach dem vorhergehenden Anspruch, **dadurch gekennzeichnet, dass** das nichtflüchtige Öl in einer Menge von 0,1 bis 50 Gew.-% und vorzugsweise 0,1 bis 30 Gew.-%, bezogen auf das Gesamtgewicht der Zusammensetzung, enthalten ist.

52. Zusammensetzung nach einem der vorhergehenden Ansprüche, **dadurch gekennzeichnet, dass** sie einen grenzflächenaktiven Stoff enthält.

53. Zusammensetzung nach einem der vorhergehenden Ansprüche, **dadurch gekennzeichnet, dass** sie einen Zusatzstoff enthält, der unter den Farbmitteln, Antioxidantien, Füllstoffen, Konservierungsmitteln, Parfums, Neutralisationsmitteln, Verdickungsmitteln, kosmetischen Wirkstoffen, Sonnenschutzfiltern, Koaleszenzmitteln und Weichmachern ausgewählt ist.

54. Zusammensetzung nach einem der vorhergehenden Ansprüche, **dadurch gekennzeichnet, dass** es sich um eine Mascara handelt,

55. Verfahren zum Schminken oder für die nichttherapeutische Pflege von Keratinfasern, insbesondere Wimpern, das das Aufbringen einer Zusammensetzung nach einem der Ansprüche 1 bis 54 auf die Keratinfasern umfasst.

56. Verwendung einer Zusammensetzung zum Überziehen von Keratinfasern nach einem der Ansprüche 1 bis 54, um Keratinfasern und insbesondere Wimpern zu krümmen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2792190 A
- EP 749747 A
- EP 749746 A
- EP 923928 A
- EP 930060 A
- FR 2232303 A
- EP 847752 A

**Littérature non-brevet citée dans la description**

- Encyclopedia of Chemical Technology, KIRK-OTHMER. WILEY, 1979, vol. 22, 333-432